(19) Europäisches Patentamt
European Patent Office
Office européen des brevets

(11) EP 3 028 697 B1

(12) **EUROPEAN PATENT SPECIFICATION**

(45) Date of publication and mention of the grant of the patent:
**26.12.2018 Bulletin 2018/52**

(21) Application number: **15185536.8**

(22) Date of filing: **20.11.2012**

(51) Int Cl.:
*A61K 31/352* (2006.01) *A61K 31/155* (2006.01)
*A61K 31/4439* (2006.01) *A61K 36/185* (2006.01)
*A61K 45/06* (2006.01) *A61P 3/10* (2006.01)

(54) **TETRAHYDROCANNABIVARIN (THCV) FOR USE IN THE PROTECTION OF PANCREATIC ISLET CELLS**

TETRAHYDROCANNABIVARIN (THCV) ZUR VERWENDUNG BEIM SCHUTZ VON PANKREASINSELZELLEN

TETRAHYDROCANNABIVARINE (THCV) POUVANT ETRE UTILISEE POUR PROTEGER LES CELLULES DES ILOTS DE LANGERHANS DU PANCREAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.11.2011 GB 201120066**

(43) Date of publication of application:
**08.06.2016 Bulletin 2016/23**

(62) Document number(s) of the earlier application(s) in accordance with Art. 76 EPC:
**12795839.5 / 2 782 568**

(73) Proprietor: **GW Pharma Limited**
**Histon**
**Cambridge CB24 9BZ (GB)**

(72) Inventors:
- **CAWTHORNE, Michael**
  **Buckingham, MK18 1EG (GB)**
- **STOTT, Colin**
  **Cambridge, CB24 9BZ (GB)**
- **WRIGHT, Stephen**
  **Cambridge, CB24 9BZ (GB)**

(74) Representative: **HGF Limited**
**4th Floor**
**Merchant Exchange**
**17-19 Whitworth Street West**
**Manchester M1 5WG (GB)**

(56) References cited:
**WO-A1-2009/093018 WO-A2-2006/054057**
**AT-A1- 509 000**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 028 697 B1

## Description

The present invention relates to the phytocannabinoid tetrahydrocannabivarin (THCV) for use in the protection of pancreatic islet cells. Preferably the pancreatic islet cells to be protected are beta cells. More preferably the protection of the pancreatic islet cells maintains insulin production at levels which are able to substantially control or improve control of blood glucose levels in a patient.

## DEFINITIONS

In this specification the following terms are used and are intended to have the following meanings / definitions:

"Cannabinoids" are a group of compounds including the endocannabinoids, the phytocannabinoids and those which are neither endocannabinoids or phytocannabinoids, hereafter "syntho-cannabinoids".

"Endocannabinoids" are endogenous cannabinoids, which are high affinity ligands of CB1 and CB2 receptors.

"Phytocannabinoids" are cannabinoids that originate in nature and can be found in the cannabis plant. The phytocannabinoids can be present in an extract including a botanical drug substance, isolated, or reproduced synthetically.

"Syntho-cannabinoids" are those compounds capable of interacting with the cannabinoid receptors (CB1 and / or CB2) but are not found endogenously or in the cannabis plant. Examples include WIN 55212 and rimonabant.

An "isolated phytocannabinoid" is one which has been extracted from the cannabis plant and purified to such an extent that all the additional components such as secondary and minor cannabinoids and the non-cannabinoid fraction have been removed.

A "synthetic cannabinoid" is one which has been produced by chemical synthesis this term includes modifying an isolated phytocannabinoid, by for example forming a pharmaceutically acceptable salt thereof.

A "botanical drug substance" or "BDS" is defined in the Guidance for Industry Botanical Drug Products Draft Guidance, August 2000, US Department of Health and Human Services, Food and Drug Administration Centre for Drug Evaluation and Research as: "A drug derived from one or more plants, algae, or microscopic fungi. It is prepared from botanical raw materials by one or more of the following processes: pulverisation, decoction, expression, aqueous extraction, ethanolic extraction or other similar processes." A botanical drug substance does not include a highly purified or chemically modified substance derived from natural sources. Thus, in the case of cannabis, BDS derived from cannabis plants do not include highly purified Pharmacopoeial grade cannabinoids.

In the present invention a BDS is considered to have two components: the phytocannabinoid-containing component and the non-phytocannabinoid containing component. Preferably the phytocannabinoid-containing component is the larger component comprising greater than 50% (w/w) of the total BDS and the non-phytocannabinoid containing component is the smaller component comprising less than 50% (w/w) of the total BDS.

The amount of phytocannabinoid-containing component in the BDS may be greater than 55%, through 60%, 65%, 70%, 75%, 80% to 85% or more of the total extract. The actual amount is likely to depend on the starting material used and the method of extraction used.

The "principle phytocannabinoid" in a BDS is the phytocannabinoid that is present in an amount that is higher than that of the other phytocannabinoids. Preferably the principle phytocannabinoid is present in an amount greater than 40% (w/w) of the total extract. More preferably the principle phytocannabinoid is present in an amount greater than 50% (w/w) of the total extract. More preferably still the principle phytocannabinoid is present in an amount greater than 60% (w/w) of the total extract.

The amount of the principle phytocannabinoid in the BDS is preferably greater than 50% of the phytocannabinoid-containing fraction, more preferably still greater than 55% of the phytocannabinoid-containing fraction, and more preferably still greater than 60% through 65%, 70%, 75%, 80%, 85%, 90% and 95% of the phytocannabinoid-containing fraction.

The "secondary phytocannabinoid/s" in a BDS is the phytocannabinoid/s that is / are present in significant proportions. Preferably the secondary phytocannabinoid is present in an amount greater than 5% (w/w) of the total extract, more preferably greater than 10% (w/w) of the total extract, more preferably still greater than 15% (w/w) of the total extract. Some BDS's will have two or more secondary phytocannabinoids that are present in significant amounts. However not all BDS's will have a secondary phytocannabinoid.

The "minor phytocannabinoid/s" in a BDS can be described as the remainder of all the phytocannabinoid components once the principle and secondary phytocannabinoids are accounted for. Preferably the minor phytocannabinoids are present in total in an amount of less than 5% (w/w) of the total extract, and most preferably the minor

phytocannabinoid is present in an amount less than 2% (w/w) of the total extract.

The term "consisting essentially of" is limited to the phytocannabinoids which are specified, it does not exclude non-cannabinoid components that may also be present.

Typically the non-phytocannabinoid containing component of the BDS comprises terpenes, sterols, triglycerides, alkanes, squalenes, tocopherols and carotenoids.

These compounds may play an important role in the pharmacology of the BDS either alone or in combination with the phytocannabinoid.

The "terpene fraction" may be of significance and can be broken down by the type of terpene: monoterpene or sesquiterpene. These terpene components can be further defined in a similar manner to the cannabinoids.

The amount of non-phytocannabinoid containing component in the BDS may be less than 45%, through 40%, 35%, 30%, 25%, 20% to 15% or less of the total extract. The actual amount is likely to depend on the starting material used and the method of extraction used.

The "principle monoterpene/s" in a BDS is the monoterpene that is present in an amount that is higher than that of the other monoterpenes. Preferably the principle monoterpene/s is present in an amount greater than 20% (w/w) of the total terpene content. More preferably the principle monoterpene is present in an amount greater than 30% (w/w) of the total terpene content, more preferably still greater than 40% (w/w) of the total terpene content, and more preferably still greater than 50% (w/w) of the total terpene content. The principle monoterpene is preferably a myrcene or pinene. In some cases there may be two principle monoterpenes. Where this is the case the principle monoterpenes are preferably a pinene and / or a myrcene.

The "principle sesquiterpene" in a BDS is the sesquiterpene that is present in an amount that is higher than all the other sesquiterpenes. Preferably the principle sesquiterpene is present in an amount greater than 20% (w/w) of the total terpene content, more preferably still greater than 30% (w/w) of the total terpene content. The principle sesquiterpene is preferably a caryophyllene and / or a humulene.

The sesquiterpene components may have a "secondary sesquiterpene". The secondary sesquiterpene is preferably a caryophyllene, which is preferably present at an amount greater than 5% (w/w) of the total terpene content, more preferably the secondary sesquiterpene is present at an amount greater than 10% (w/w) of the total terpene content.

The secondary sesquiterpene is preferably a humulene which is preferably present at an amount greater than 5% (w/w) of the total terpene content, more preferably the secondary sesquiterpene is present at an amount greater than 10% (w/w) of the total terpene content.

Alternatively botanical extracts may be prepared by introducing isolated phytocannabinoids or their synthetic equivalent into a non-cannabinoid plant fraction as can be obtained from a zero cannabinoid plant or one or more non-cannabinoid components found in the cannabis plant such as terpenes.

The structure of the phytocannabinoid THCV is shown below:

| | | |
|---|---|---|
| THCV | Tetrahydrocannabivarin | 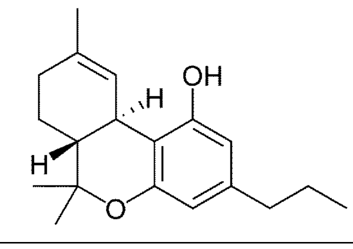 |

Phytocannabinoids can be found as either the neutral (decarboxylated form) or the carboxylic acid form depending on the method used to extract the cannabinoids. For example it is known that heating the carboxylic acid form will cause most of the carboxylic acid form to decarboxylate into the neutral form.

Where a synthetic phytocannabinoid is used the term is intended to include compounds, metabolites or derivatives thereof, and pharmaceutically acceptable salts of such compounds.

The term "pharmaceutically acceptable salts" refers to salts or esters prepared from pharmaceutically acceptable non-toxic bases or acids, including inorganic bases or acids and organic bases or acids, as would be well known to persons skilled in the art. Many suitable inorganic and organic bases are known in the art.

For the purpose of this invention the term "treatment" is intended to encompass protection of the pancreatic islet cells and a therapeutically effective amount of THCV is an amount that provides a degree of protection.

## BACKGROUND TO THE INVENTION

The pancreas is a gland in the digestive system of vertebrates and produces important hormones including

insulin, glucagon and somatostatin as well as being a digestive organ secreting digestive enzymes to assist in the absorption of nutrients.

The pancreas comprises two different types of tissue; the islets of Langerhans which produce and secrete hormones including insulin and the pancreatic acini which produce and secrete digestive enzymes.

Four main cell types exist in the islets: alpha cells secrete glucagon, which increases glucose concentration in the blood; beta cells secrete insulin, which decrease glucose in the blood; delta cells secrete somatostatin, which regulates alpha and beta cells; and PP cells which secrete pancreatic polypeptide.

The islets of Langerhans play an imperative role in glucose metabolism and regulation of blood glucose concentration.

Diabetes mellitus is a disease that is caused by either or both deficiency or diminished effectiveness of the insulin which is produced by the pancreatic islets. It is characterised by hyperglycemia, unbalanced metabolism and other conditions predominantly affecting the vascular structures.

The main types of diabetes mellitus are: Type 1 diabetes mellitus, which results from the body's failure to produce sufficient insulin and Type 2 diabetes mellitus which results from resistance to insulin, often initially with normal or increased levels of circulating insulin, though ultimately a failure to produce sufficient insulin.

Type 1 diabetes mellitus is a form of diabetes that results from autoimmune destruction of insulin-producing beta cells of the pancreas. The subsequent lack of insulin leads to increased blood glucose and urinary excretion of glucose.

Other types of diabetes mellitus include: Gestational diabetes where pregnant women who have never had diabetes before but who have high blood sugar (glucose) levels during pregnancy develop diabetes. This type of diabetes affects about 4% of all pregnant women. It may precede development of type 2 diabetes mellitus.

Secondary diabetes: accounts for approximately 1-2% of patients with diabetes mellitus. Causes include: Pancreatic diseases such as cystic fibrosis, chronic pancreatitis, pancreatectomy, carcinoma of the pancreas; Endocrine diseases such as Cushing's syndrome, acromegaly, thyrotoxicosis, phaeochromocytoma, glucagonoma; Drug-induced diabetes such as thiazide diuretics, corticosteroids, atypical antipsychotics, antiretroviral protease inhibitors; congenital lipodystrophy; Acanthosis nigricans; and genetic causes such as Wolfram syndrome, Friedreich's ataxia, dystrophia myotonica, haemochromatosis, and glycogen storage diseases.

Some patients with type 2 diabetes require insulin so the old terms of insulin-dependent diabetes mellitus for type 1 diabetes and non-insulin dependent diabetes mellitus for type 2 diabetes are inappropriate.

Type 1 diabetes mellitus accounts for less than 15% of diabetics. It is usually a juvenile onset disease but may occur at any age. It may be associated with other autoimmune diseases and is characterised by insulin deficiency.

The cause of type 1 diabetes mellitus is unknown however it has been discovered that a gene determines islet sensitivity to damage from viruses.

Patients with type 1 diabetes mellitus always need insulin treatment and are prone to ketoacidosis. Diabetic ketoacidosis occurs when the body cannot use glucose as a fuel source because there is no insulin or not enough insulin. Fat is used for fuel instead and the byproducts of fat breakdown, called ketones, build up in the body.

Symptoms of ketoacidosis include: deep, rapid breathing; dry skin and mouth; flushed face; fruity smelling breath; nausea, vomiting and stomach pain. If the ketoacidosis is not treated decreased consciousness may occur which may worsen to a coma or even death.

Patients with type 2 diabetes mellitus account for more than 85% of all cases of diabetes mellitus. Type 2 diabetics are usually older at presentation (>30 years of age) but this disease is increasingly being diagnosed in children and adolescents.

Type 2 diabetes is often associated with excess body weight and physical inactivity and is caused by impaired insulin secretion and insulin resistance. These two defects interact. Thus to combat insulin resistance, the islet cells produce more insulin but over time this overproduction results in further compromising islet cell integrity. At the time of diagnosis of diabetes, pancreatic islet cell mass will have been reduced by at least 50%. Type 2 diabetes mellitus has a gradual onset and may eventually require insulin treatment.

Metabolic syndrome is thought of as a precursor to type 2 diabetes. This syndrome is poorly defined and represents a heterogeneous collection of various propensities to diabetes. It has been suggested that lifestyle-intervention and treating metabolic manifestations of this pre-diabetic state can reduce the chance of progression to frank diabetes and the risk of complications.

The aims of current treatment are the avoidance of complications. Strict plasma glucose control reduces renal, neurological and retinal damage. A balance is required for each patient between low blood glucose readings and the risk of hypoglycemia.

The prognosis has improved considerably with the development of insulin therapies although many diabetics develop blindness, end-stage renal disease and, in some cases, early death. Controlling blood glucose, lipids, blood pressure and weight are important factors and predict the development of long-term macrovascular and microvascular complications.

Mortality is two to three times higher among people with type 2 diabetes than in the general population. Indeed 75% of people with type 2 diabetes die of heart disease and 15% of stroke. The mortality rate from cardiovascular disease is up to five times higher in people with diabetes than in people without diabetes.

Treatment goals for type 1 diabetes mellitus is to minimize any elevation of blood sugar (glucose) without causing abnormally low levels of blood sugar. Type 1 diabetes mellitus is therefore treated with insulin, exercise, and dietary modification.

Type 2 diabetes mellitus is treated first with weight reduction, dietary modification, and exercise. When these measures fail to control the elevated blood sugars, oral medications are used. If oral medications are still insufficient, treatment with insulin or other medications are considered.

Several different types of medicine can be used to treat type 2 diabetes; a combination of two or more medicines may be required to control the blood glucose level. Many provide merely symptomatic relief such as weight reduction, others provide disease modifying effects.

Typical anti-diabetic drugs include: Metformin; Sulphonylureas; Glitazones; Gliptins; GLP-1 agonists; Acarbose; Nateglinide and repaglinide. SGLT-2 inhibitors are also being developed.

Metformin is often the first medicine that is recommended to treat type 2 diabetes. It works by reducing the amount of glucose that the liver releases into the bloodstream. It also makes the body's cells more responsive to insulin. Side effects of metformin include nausea, vomiting and diarrhea.

Sulphonylureas increase the amount of insulin that is produced by the pancreas. Examples of sulphonylureas include: glibenclamide; gliclazide; glimerpirizide; glipizide; and gliquidone. Sulphonylureas can increase the risk of hypoglycaemia (low blood glucose) because they increase the amount of circulating insulin. Sulphonylureas may cause other side effects including weight gain, nausea and diarrhea.

Glitazones such as thiazolidinedione medicines (pioglitazone) make cells more sensitive to insulin so that more glucose is taken from the blood. They are not often used alone, but are usually used in addition to metformin or sulphonylureas, or both. They may cause weight gain and water retention. Another thiazolidinedione, rosiglitazone, has been withdrawn from use because of the increased risk of cardiovascular disorders, including heart attack and heart failure.

Gliptins are DPP-4 inhibitors which work by inhibiting the breakdown of a naturally occurring hormone called GLP-1. GLP-1 helps the body produce insulin in response to high blood glucose levels, but is rapidly broken down. By preventing this breakdown, the gliptins (such as sitagliptin and vildagliptin) act to prevent high blood glucose levels, but do not result in episodes of hypoglycaemia.

GLP-1 agonists such as Exenatide are an injectable treatment that acts in a similar way to the natural hormone GLP-1 but have longer plasma half-lives. They are injected twice a day and boosts insulin production when there are high blood glucose levels, reducing blood glucose without the risk of hypoglycaemic episodes. They also lead to modest weight loss in many people who take them. They are mainly used in people on metformin and/or sulphonylurea who are obese (with a BMI of 35 or above).

Acarbose helps prevent blood glucose level from increasing too much after a meal. It slows down the rate at which the digestive system breaks down carbohydrates into glucose. Acarbose is not often used to treat type 2 diabetes because it usually causes side effects, such as bloating, diarrhoea and meteorism.

Nateglinide and repaglinide stimulate the release of insulin by the pancreas. They are not commonly used but may be an option if meals are eaten at irregular times. This is because their effects do not last very long, but they are effective when taken just before a meal. Nateglinide and repaglinide can cause side effects, such as weight gain and hypoglycaemia (low blood glucose).

The patent GB 2434097 discusses the properties of THCV. The patent describes receptor binding studies which show that THCV is a CB-1 receptor neutral antagonist, as such the patent paragraphs using THCV to treat conditions benefitting from neutral antagonism of the CB-1 receptor. Such conditions include obesity, schizophrenia, epilepsy and obesity associated with type 2 diabetes (a symptomatic effect of type 2 diabetes).

US 2007/0099987 discusses the use of the cannabinoid cannabidiol (CBD) in the prevention or treatment of type 1 diabetes and / or insulitis.

WO 2009/007697 describes a pharmaceutical formulation which comprises a ratioed mix of the cannabinoids THCV and CBD based on the pharmacology of the respective compounds.

WO 2009/093018 discusses the use of a combination of CBD and THCV to manage or treat metabolic syndrome or a cluster of disorders which commonly occur together including: type I or type II diabetes, obesity, dyslipidemia, or cardiovascular disease. It is described that these conditions are managed or treated by controlling cholesterol levels (a CBD effect) and increasing energy expenditure in a subject (a THCV effect).

WO 2007/032962 describes an intranasal formulation comprising tricyclic cannabinoids. Whilst a THCV formulation is envisaged there is no specific disclosure of the use of this compound for treatment of a particular disorder. All exemplification relates specifically to the use of THC.

WO2006/054057 teaches the use of THCV as a CB1 receptor antagonist for the treatment of obesity associated with type II diabetes. The technical effect is reduction of appetite.

Indeed none of the above documents teach or suggest that THCV can be used alone or in combination to protect the pancreatic islet cells and thereby maintain insulin function. This protective effect enables a formulation comprising or consisting of THCV to be used to treat in a disease modifying manner (as opposed to providing symptomatic relief such as weight reduction or blood glucose control), diseases such as diabetes. Thus in addition to treating type 1 diabetes it is also possible to more effectively manage type 2 diabetes and treat pre-diabetic patients to prevent the onset of diabetes and other metabolic related conditions.

A critical issue when treating human subjects is the evaluation of islet β-cell mass and function in response to treatment. A significant limitation of interventional trials in humans is that there are no "gold standard" methods to directly measure β-cell mass *in vivo.* Newer imaging techniques like positron emission tomography, magnetic resonance imaging, scintigraphy, or neurofunctional imaging approach are undergoing development as non-invasive methods of islet β-cell mass measurement.

Metabolic tests have been routinely used as surrogate markers, and studies have shown that acute insulin response to arginine, glucose and glucose-potentiated and arginine-induced insulin secretion can be used as tests for estimation of islet β-cell mass. A well-validated and practical means of quantifying insulin secretion *in vivo* is measurement of C-peptide levels under standardized conditions, which has low variability and high reproducibility, making it a good and reliable marker. In fact, the recommendation of an expert panel convened by the American Diabetes Association was that C-peptide response (CPR) is the most appropriate measure of function and clinical end point of intervention in human clinical trials.

Type 2 diabetes is associated with insulin resistance and reduced insulin secretion, which results in hyperglycaemia. This can then lead to diabetic complications such as retinopathy, neuropathy, nephropathy and cardiovascular disease. Although insulin resistance may be present earlier in the progression of the disease, it is now generally accepted that it is the deterioration in insulin-secretory function that leads to hyperglycaemia.

This reduction in insulin secretion in type 2 diabetes is due to both islet β-cell dysfunction and death.

Interventions that maintain the normal function and protect the pancreatic islet β-cells from death are crucial in the treatment of type 2 diabetes so that fasting plasma glucose levels may be maintained within or approaching the normal range (between about 3.6 and 5.8 mM, or 64.8 and 104.4 mg/dL). Compounds which are found to protect islet β-cells from failure and increase or prevent the decrease of islet β-cell mass are crucial in the treatment of type 2 diabetes.

A fasting serum insulin level of greater than approximately 60 pmol/L is considered evidence of insulin resistance. Therefore maintaining fasting insulin levels at or near to this level is desirable.

A glucose tolerance test (GTT) is often used to diagnose diabetes. A fasting patient takes a 75 gram oral dose of glucose. Blood glucose levels are then measured over the following 2 hours. After 2 hours a glycaemia less than 7.8 mmol/L is considered normal, a glycaemia of between 7.8 to 11.0 mmol/dl is considered as impaired glucose tolerance and a glycaemia of greater than or equal to 11.1 mmol/dl (200 mg/dl) is considered to be diabetes mellitus

Insulin resistance is often measured using the hyperinsulinemic euglycaemic clamp. This is the gold standard for investigating and quantifying insulin resistance. It measures the amount of glucose necessary to compensate for an increased insulin level without causing hypoglycemia.

Insulin is infused at 10-120 mU per $m^2$ per minute. Glucose 20% is infused to maintain blood sugar levels between 5 and 5.5 mmol/l. The rate of glucose infusion is determined by checking the blood sugar levels every 5 to 10 minutes. Low-dose insulin infusions are more useful for assessing the response of the liver, whereas high-dose insulin infusions are useful for assessing peripheral (i.e., muscle and fat) insulin action.

The rate of glucose infusion during the last 30 minutes of the test determines insulin sensitivity. If high levels (7.5 mg/min or higher) are required, the patient is insulin-sensitive. Very low levels (4.0 mg/min or lower) indicate that the body is resistant to insulin action. Levels between 4.0 and 7.5 mg/min are not definitive and suggest "impaired glucose tolerance," an early sign of insulin resistance.

It is an object of the present invention to provide a means of protecting the pancreatic islet cells. In providing such protection of the islets conditions which are caused by damage or dysfunction of the islets such as diabetes mellitus can be treated at an earlier stage or a different treatment strategy can be employed.

However, surprisingly it has been observed in a rodent model of diabetes, that THCV (CB1 neutral antagonist) is able to protect the insulin-producing cells of the pancreatic islets and give rise to a reduction in fasting insulin indicative of improved insulin resistance. Islet cell preservation is seen as a highly desirable feature of a new anti-diabetic medicine.

## BRIEF SUMMARY OF THE DISCLOSURE

According to the present invention there is provided the phytocannabinoid tetrahydrocannabivarin (THCV) for use as an oral anti-diabetic medication, wherein the patient has or is pre-disposed to type 1 diabetes or is predisposed to gestational diabetes.

In one embodiment the THCV is used in combination with one or more additional anti-diabetic medicines and / or one or more anti-obesity medicines. Preferably the additional anti-diabetic medicine is metformin or is from the

sulphonylurea class of anti-diabetic drugs.

Other anti-diabetic medications which could be used include: glitazones; gliptins; GLP-1 agonists; acarbose; nateglinide, and SGLT-2 inhibitors. Other anti-diabetic medications are being developed.

In a further embodiment the THCV is a synthetic cannabinoid or an isolated phytocannabinoid.

In a separate embodiment the THCV is present as an extract from a cannabis plant. Preferably the extract from a cannabis plant is a botanical drug substance. More preferably the extract is substantially free of the phytocannabinoids tetrahydrocannabinol (THC) and / or cannabidiol (CBD).

A typical THCV BDS is as described in Tables 1.1 and 1.2 below:

**Table 1.1 Tetrahydrocannabivarin BDS amount in total and range**

| THCV BDS | Amount (% w/w) | Range (± 10%) | Range (± 25%) | Range (± 50%) |
|---|---|---|---|---|
| CBGV | 0.15 | 0.14 - 0.17 | 0.11 - 0.19 | 0.07 - 0.23 |
| CBNV | 1.30 | 1.20 - 1.40 | 1.00 - 1.60 | 0.65 - 1.95 |
| **THCV** | **64.49** | **58.04 - 70.94** | **48.37 - 80.61** | **32.25 - 96.74** |
| CBCV | 0.65 | 0.59 - 0.72 | 0.49 - 0.81 | 0.33 - 0.98 |
| THC-C4 | 0.82 | 0.74 - 0.90 | 0.62 - 1.03 | 0.41 - 1.23 |
| CBN | 0.15 | 0.14 - 0.17 | 0.11 -0.19 | 0.07 - 0.23 |
| THCVA | 0.36 | 0.32 - 0.40 | 0.27 - 0.45 | 0.18 - 0.54 |
| THC | 13.43 | 12.09 - 14.77 | 10.07 - 16.79 | 7.72 - 20.15 |
| Unknowns | 0.58 | 0.52 - 0.64 | 0.44 - 0.73 | 0.29 - 0.87 |
| **Total Cannabinoids** | **81.93** | | | |
| **Total Non-cannabinoids** | **18.07** | | | |

The total phytocannabinoid containing fraction of THCV BDS comprises approximately 74-90% (w/w) of the total BDS.

**Table 1.2 Tetrahydrocannabivarin BDS by percentage cannabinoid**

| THCV BDS | Amount (% of total cannabinoid) |
|---|---|
| CBGV | 0.18 |
| CBNV | 1.59 |
| **THCV** | **78.71** |
| CBCV | 0.79 |
| THC-C4 | 1.00 |
| CBN | 0.18 |
| THCVA | 0.44 |
| THC | 16.39 |
| Unknowns | 0.71 |

The amount of the principle phytocannabinoid in the THCV BDS as a percentage of the phytocannabinoid containing fraction is approximately 71-87% (w/w). The THCV BDS also has a secondary cannabinoid THC which is present at approximately 14.8-18% (w/w) of the phytocannabinoid containing fraction.

The THCV is present in a therapeutically acceptable amount, which may, for example, be between 1mg and 2000mg.

The human dose equivalent (HED) can be estimated using the following formula:

$$\text{HED} = \text{Animal dose (mg/kg)}\ \textit{multiplied by}\ \frac{\text{Animal } K_m}{\text{Human } K_m}$$

The $K_m$ for a mouse is 3 and the $K_m$ for a human is 37.

In accordance with a second aspect of the present invention there is provided the phytocannabinoid tetrahydrocannabivarin (THCV) for use in the treatment of type 1 diabetes.

In accordance with a third aspect of the present invention there is provided the phytocannabinoid tetrahydrocannabivarin (THCV) for use as an oral anti-diabetic medication.

In accordance with a fourth aspect of the present invention there is provided the phytocannabinoid tetrahydrocannabivarin (THCV) for use as a GLP-1 agonist.

**BRIEF DESCRIPTION OF THE DRAWINGS**

Embodiments of the invention are further described hereinafter with reference to the accompanying drawings, in which:

Figure 1 shows a photograph of stained pancreatic islet cell prior to treatment;

Figure 2 shows a photograph of stained pancreatic islet cell in vehicle treated diabetic mouse;

Figure 3 shows a photograph of stained pancreatic islet cell in AM251 treated diabetic mouse;

Figure 4 shows a photograph of stained pancreatic islet cell in CBD treated diabetic mouse;

Figure 5 shows a photograph of stained pancreatic islet cell in THCV treated diabetic mouse;

Figure 6 shows the bodyweight change of the animals over the study period;

Figure 7 shows the blood glucose concentration of the animals over a 24 hour period;

Figure 8 shows the pancreatic islet beta cell mass in animals at the end of the study period.

Figure 9 shows the concentration of blood glucose of animals at day 0;

Figure 10 shows the concentration of blood glucose of animals at day 7;

Figure 11 shows the concentration of blood glucose of animals at day 14;

Figure 12 shows the concentration of blood glucose of animals at day 23;

Figure 13 shows the area under the curve after animals were given an oral glucose tolerance test at day 31;

Figure 14 shows the mean change from baseline of the concentration of insulin during an oral glucose tolerance test in the THCV treated group at Visit 5;

Figure 15 shows the mean change from baseline of the concentration of blood glucose during an oral glucose tolerance test in the THCV treated group at Visit 5;

Figure 16 shows the change from baseline in HOMA2 insulin sensitivity;

Figure 17 shows the change from baseline in HOMA2 beta cell function; and

Figure 18 shows the change from baseline in mean serum glucagon-like peptide 1 (GLP-1) levels.

**DETAILED DESCRIPTION**

The invention is illustrated by way of the following Examples.

Example 1 was designed to examine whether cannabidiol (CBD) and/or tetrahydrocannabivarin (THCV) are able to affect pancreatic islet cell morphology.

Example 2 looked at the effect of THCV in combination with an anti-diabetic medicament, exemplified by rosiglitazone.

Example 3 demonstrates the use of THCV in a clinical study whereby the effect of this phytocannabinoid in man is demonstrated for the first time.

## EXAMPLE 1: EFFECT OF TETRAHYDROCANNABIVARIN (THCV) AND CANNABIDIOL (CBD) ON ISLET CELL MORPHOLOGY AND FUNCTION IN DIABETIC MICE

### Materials and Methods

The animals used in this study were male *db/db* mice which were aged 7 to 8 weeks on commencement of the study. The *db/db* mouse is a model of obesity, diabetes, and dyslipidemia. The mice were obtained from Charles River (Italy) and fed on the Beekay Rat and Mouse Diet Number 1 throughout the study.

The animals were weighed and grouped into 8 animals per group, 4 animals per cage and dosed as described in Table 1.3 below:

**Table 1.3 Dosing Groups**

| GROUP | Dose |
|---|---|
| A | 10 ml/kg Vehicle |
| B | 10 mg/kg THCV |
| C | 10 mg/kg AM 251 |
| D | 10 mg/kg CBD |
| E | Baseline measurements |

The phytocannabinoids CBD (10mg/kg) and THCV (10mg/kg) were tested along with AM 251 (10mg/kg) which was used as a positive control.

At the start of the study the Group E animals were sacrificed and a terminal blood sample was taken. In addition four of the animals pancreases were sampled for determination of β-cell area, islet size and β-cell mass by immunoblotting.

On day 1 dosing commenced for groups A to D as outlined in Table 1.1 above. Animals were dosed daily at 17:00.

Throughout the 28 day study the animals in each group were weighed and blood samples were taken for glucose, insulin, triglycerides, HDL and total cholesterol.

On day 25 of the study the body composition of the animals was measured using DEXA scanning.

At the end of the study the animals were sacrificed and a terminal blood sample was taken. In addition four of the animals pancreases were sampled for determination of islet β-cell area, islet size and islet β-cell mass by immunoblotting.

The pancreases were stained for insulin and the pancreatic islet cells were examined under a microscope to determine the amount of insulin in the cells.

### Results

The histology findings indicated that THCV caused a greater retention of insulin in the pancreatic islet than both AM251 and CBD. This finding suggests that the phytocannabinoid is islet cell protective.

Figure 1 illustrates the pancreatic islets in the untreated group and Figures 2 to 5 illustrate photographs of the stained pancreatic islet cells from the different treatment groups. As can be observed in Figure 5 the islets in the animals treated with the THCV are far darker than those in the CBD, AM251 and vehicle groups indicating that there is statistically more insulin present in the pancreatic islets.

Figure 6 shows that the animals treated with the CBD had an increased weight gain, however the weight gain of the animals treated with the THCV and AM 251 was similar to controls and indeed food intake was slightly lower.

Figure 7 demonstrates that the blood glucose concentration of the animals treated with THCV was more controlled during a 24 hour period. In effect there were no period of hyperglycaemia or hypoglycaemia which is indicative of stable blood glucose control.

Figure 8 illustrates from morphological analysis that the pancreatic beta cell mass was higher in the THCV treated animals than in the vehicle and CBD and AM251 treated groups.

**Conclusion**

The data above demonstrate that THCV is able to induce islet cell protection in diabetic mice without having a profound reduction in blood glucose.
This finding is of real significance and leads to the conclusion that the phytocannabinoid THCV is islet cell protective and as such a significant treatment option for diabetes.

**EXAMPLE 2: EFFECT OF TETRAHYDROCANNABIVARIN (THCV) AND ROSIGLITAZONE ON PLASMA GLUCOSE LEVELS IN DIABETIC MICE**

**Materials and Methods**

The animals used in this study were male *db*/*db* mice which were aged 7 to 8 weeks on commencement of the study. The *db*/*db* mouse is a model of obesity, diabetes, and dyslipidemia. The mice were obtained from Charles River (Italy) and fed on the Beekay Rat and Mouse Diet Number 1 throughout the study.
The animals were weighed and grouped into 8 animals per group, 4 animals per cage and dosed as described in Table 1.4 below:

**Table 1.4 Dosing Groups**

| GROUP | Dose |
|---|---|
| A | 10 ml/kg Vehicle |
| B | 10 mg/kg THCV |
| C | 10 mg/kg Rosiglitazone |
| D | 10 mg/kg Sitagliptin |
| E | 10 mg/kg THCV + 10 mg/kg Rosiglitazone |

Sitagliptin is an anti-diabetic drug and was used as a positive control.
On day 1 dosing commenced for groups A to E as outlined in Table 1.4 above. Animals were dosed daily at 17:00.
At set time periods: day 0, day 7, day 14, and day 23, throughout the study the animals in each group were weighed and blood samples were taken for analysis.

**Results**

Figures 9 to 12 illustrate the blood glucose level of the animals in each group at the different time periods. As can be seen the blood glucose level decreases over the study period in the group treated with the combination on THCV and rosiglitazone and statistically significant data is obtained for this group at all time points.
Figure 13 demonstrates the area under the curve during an oral glucose tolerance test on day 31. These data show that using Bonferroni's Multiple Comparison Test the vehicle versus the combination of THCV and Rosiglitazone was shown to be significant.

**Conclusion**

The combination of THCV with anti-diabetic medications produces a significant reduction in blood glucose. This ability of THCV to reduce the blood glucose level in diabetic animals provides further evidence for its use either alone or in combination with other anti-diabetic drugs in the treatment of diabetes.

**EXAMPLE 3: A RANDOMISED, DOUBLE BLIND, PLACEBO CONTROLLED, PARALLEL GROUP, PILOT STUDY OF 1:1 AND 20:1 RATIO OF FORMULATED CBD:THCV PLUS CBD AND THCV ALONE IN THE TREATMENT OF DYSLIPIDAEMIA IN SUBJECTS WITH TYPE 2 DIABETES**

The aim of the pilot study was to evaluate the treatment of dyslipidaemia in subjects with Type 2 diabetes who

have failed to achieve satisfactory lipid control with existing treatments.

**Materials and Methods**

There were four arms in this study plus a placebo comparator. These were a 1:1 and 20:1 ratio of CBD:THCV, CBD alone and THCV alone. Assessment of the impact of each treatment on different parameters was made. Measurements were taken of high density lipoprotein (HDL) cholesterol, total cholesterol, low density lipoprotein (LDL) cholesterol, HDL / LDL ratio, serum triglycerides, apolipoprotein markers (Apo A & Apo B) and determination of ApoA/Apo B ratio.

Other measurements including: lipid parameters; glucose control (fasting plasma glucose, glucose tolerance, serum fructosamine, glycosylated haemoglobin A1c (HbA1c) (whole blood)); Insulin sensitivity (insulin resistance); body weight & body mass index (BMI); adipose tissue distribution (total % body fat content, waist circumference, neck circumference, waist-to-hip ratio, visceral adiposity, liver triglyceride content); and appetite 11 point numerical rating scale (0-10 NRS).

The safety and tolerability of the test compounds compared with placebo were also assessed measurements were recorded for: adverse events (AE); vital signs; Beck Depression Inventory (BDI); Electrocardiogram (ECG).

Laboratory assessments included; physical examination; markers of vascular function; markers of adipocyte function including leptin and adiponectin; markers of inflammation including cytokines; retinol binding protein (RBP4) concentration; orexin type A (Orexin A) concentration; gut signalling hormone (Gastric Inhibitory Peptide (GIP), Glucagon-like peptide - 1 (GLP-1), ghrelin) concentrations; ketone bodies; and endocannabinoid plasma levels.

The body of data collected for the study was considerably large and as such only representative data are presented within this example.

The study took place over 15-19 weeks (1-5 week baseline and 13 week treatment period and 1 week follow-up), and was a multicentre, randomised, double blind, placebo controlled, parallel group pilot study which evaluated the test compounds on lipid parameters in subjects with Type 2 diabetes.

All subjects were receiving either metformin or a sulphonylurea medication yet they had failed to achieve satisfactory lipid and / or glucose control with their existing medication.

Eligible subjects entered the study at a Screening Visit (Visit 1, Day -35 to -7) and commenced a seven to thirty-five (7-35) day baseline period, before returning for a randomisation visit (Visit 2, Day 1).

At the discretion of the investigator (based on individual subjects), the Screening Visit (Visit 1) may have been split into two separate visits (Visits 1A and Visit 1B), to allow a 21-day washout period of prohibited medications prior to blood sampling for eligibility.

Subjects returned for a baseline visit (Visit 2, Day 1, Baseline Visit) where eligible subjects were randomised to treatment groups.

Further study visits took place at the end of Week 4 of treatment (Visit 3, Day 29), and again at the end of treatment at Week 13 (Visit 5, Day 92). Subjects were asked to fast overnight before Visits 1 (or 1B), 2 and 5 (minimum 8 hours). A telephone assessment was also performed at Day 57 (Visit 4) and at Week 14 (Visit 6, Day 99) for safety follow-up.

Diabetic and dyslipidaemic medication usage (where applicable), and NRS appetite visit data will be collected daily during the treatment period using the study diary.

As this was a pilot study, a formal sample size calculation was not required. Each treatment group consisted of 10 subjects. There was five treatment groups 1:1 and 20:1 ratio of CBD:THCV plus CBD alone, THCV alone and placebo.

There were five arms to the study which were as follows:

| Treatment group | Test article |
|---|---|
| 1 | 5 mg CBD / 5 mg THCV twice daily (1:1) |
| 2 | 100 mg CBD / 5 mg THCV twice daily (20:1) |
| 3 | 100 mg CBD twice daily |
| 4 | 5 mg THCV twice daily |
| 5 | Placebo twice daily |

**Results**

Figures 14 and 15 demonstrate the mean concentration of insulin and glucose in the test subjects treated with

THCV blood during an oral glucose tolerance test compared to placebo at the end of the treatment period (Visit 5).

As can be seen in Figure 14, the concentration of insulin in the THCV treated group increases over the first hour and then reduces down to the same level as the placebo after 2 hours.

Figure 15 demonstrates that this increase of insulin has the effect of quickly reducing the blood glucose level compared to placebo.

Figures 16 and 17 demonstrate data for all treatment groups using HOMA2 data calculations. This is a computer generated algorithm which provides data for homeostasis model assessments via a calculation of the concentrations of insulin and glucose.

Figure 16 shows the mean change from baseline at the end of the study period of the subject's insulin sensitivity. As can be seen the group treated with the THCV had an increased sensitivity to insulin. As type 2 diabetes is associated with reduced insulin secretion, which results in hyperglycaemia these data support the finding in Example 1 that THCV is protective for beta cells.

Figure 17 shows the mean change from baseline at the end of the study period of the subjects beta cell function. As can be seen the group treated with THCV had a much increased beta cell function compared to all the other groups. This result was statistically significant and also supports the conclusions from Example 1 that THCV is beta cell protective.

Figure 18 demonstrates the change from baseline in mean serum Glucagon-Like Peptide 1 (GLP-1) levels. GLP-1 is a potent anti-hyperglycemic hormone, inducing glucose-dependent stimulation of insulin secretion while suppressing glucagon secretion.

Such glucose-dependent action is particularly attractive because, when the plasma glucose concentration is in the normal fasting range, GLP-1 no longer stimulates insulin to cause hypoglycemia.

GLP-1 appears to restore the glucose sensitivity of pancreatic β-cells, it is also known to inhibit pancreatic β-cell apoptosis and stimulate the proliferation and differentiation of insulin-secreting β-cells. In addition, GLP-1 inhibits gastric secretion and motility. This delays and protracts carbohydrate absorption and contributes to a satiating effect.

GLP-1 agonists are a class of anti-diabetic medications, most of which are in the form of injectable formulations.

The data shown in Figure 18 surprisingly demonstrates that an oral dose of THCV is able to act as a GLP-1 agonist. The group treated with THCV showed the highest increase in GLP-1 suggesting that this compound is acting as a GLP-1 agonist.

### Conclusion

The data presented in this Example demonstrates that THCV is a highly useful medicament in for use in the protection of pancreatic islet cells. It also demonstrates that the compound is able to be used effectively as an oral preparation; this alone is of most importance due to many anti-diabetic medications being in the injectable form.

Furthermore THCV in this study was used in patients who were taking their existing medication of either metformin or sulphonylurea which also supports the conclusion of Example 2 that THCV is effective when used in combination with other anti-diabetic medication.

## Claims

1. The phytocannabinoid tetrahydrocannabivarin (THCV) for use as an oral anti-diabetic medication, wherein the patient has or is pre-disposed to type 1 diabetes or is pre-disposed to gestational diabetes.

2. The phytocannabinoid tetrahydrocannabivarin (THCV) for use according to claim 1, which is a disease modifying treatment as opposed to a symptomatic treatment.

3. The phytocannabinoid tetrahydrocannabivarin (THCV) for use according to any of the preceding claims, in combination with one or more additional anti-diabetic medicine and or anti-obesity medicines.

4. The phytocannabinoid tetrahydrocannabivarin (THCV) for use according to claim 3, wherein the additional anti-diabetic medicine is metformin.

5. The phytocannabinoid tetrahydrocannabivarin (THCV) for use according to claim 3, wherein the additional anti-diabetic medicine is from the sulphonylurea class of anti-diabetic drugs.

6. The phytocannabinoid tetrahydrocannabivarin (THCV) for use according to any of the preceding claims, wherein the THCV is a synthetic cannabinoid or an isolated phytocannabinoid.

7. The phytocannabinoid tetrahydrocannabivarin (THCV) for use according to any of the claims 1 to 5, wherein the THCV is present as an extract from a cannabis plant.

8. The phytocannabinoid tetrahydrocannabivarin (THCV) for use according to claim 7, wherein the extract from a cannabis plant is a botanical drug substance.

9. The phytocannabinoid tetrahydrocannabivarin (THCV) for use according to claim 7 or 8, which is substantially free of the phytocannabinoids tetrahydrocannabinol (THC) and / or cannabidiol (CBD).

10. The phytocannabinoid tetrahydrocannabivarin (THCV) for use according to any of the preceding claims, wherein the therapeutically effective dose of THCV is between 1 and 2000 mg.

## Patentansprüche

1. Phytocannabinoid Tetrahydrocannabivarin (THCV) zur Verwendung als ein orales antidiabetisches Medikament, wobei der Patient Typ-1-Diabetes hat oder dafür prädisponiert ist oder für Schwangerschaftsdiabetes prädisponiert ist.

2. Phytocannabinoid Tetrahydrocannabivarin (THCV) zur Verwendung nach Anspruch 1, bei der es sich um eine krankheitsmodifizierende Behandlung im Gegensatz zu einer symptomatischen Behandlung handelt.

3. Phytocannabinoid Tetrahydrocannabivarin (THCV) zur Verwendung nach einem der vorhergehenden Ansprüche in Kombination mit einem oder mehreren zusätzlichen antidiabetischen Arzneimitteln und/oder Arzneimitteln gegen Adipositas.

4. Phytocannabinoid Tetrahydrocannabivarin (THCV) zur Verwendung nach Anspruch 3, wobei das zusätzliche antidiabetische Arzneimittel Metformin ist.

5. Phytocannabinoid Tetrahydrocannabivarin (THCV) zur Verwendung nach Anspruch 3, wobei das zusätzliche antidiabetische Arzneimittel aus der Sulfonylharnstoff-Klasse von antidiabetischen Wirkstoffen ist.

6. Phytocannabinoid Tetrahydrocannabivarin (THCV) zur Verwendung nach einem der vorhergehenden Ansprüche, wobei das THCV ein synthetisches Cannabinoid oder ein isoliertes Phytocannabinoid ist.

7. Phytocannabinoid Tetrahydrocannabivarin (THCV) zur Verwendung nach einem der Ansprüche 1 bis 5, wobei das THCV als ein Extrakt von einer Cannabispflanze vorliegt.

8. Phytocannabinoid Tetrahydrocannabivarin (THCV) zur Verwendung nach Anspruch 7, wobei der Extrakt von einer Cannabispflanze eine botanische Wirksubstanz ist.

9. Phytocannabinoid Tetrahydrocannabivarin (THCV) zur Verwendung nach Anspruch 7 oder 8, das im Wesentlichen frei von den Phytocannabinoiden Tetrahydrocannabinol (THC) und/oder Cannabidiol (CBD) ist.

10. Phytocannabinoid Tetrahydrocannabivarin (THCV) zur Verwendung nach einem der vorhergehenden Ansprüche, wobei die therapeutisch wirksame Dosis von THCV zwischen 1 und 2000 mg liegt.

## Revendications

1. Tétrahydrocannabivarinne (THCV), un phytocannabinoïde, pour utilisation comme un médicament antidiabétique oral, dans lequel le patient a ou est prédisposé à un diabète de type 1 ou est prédisposé à un diabète gestationnel.

2. Tétrahydrocannabivarinne (THCV), un phytocannabinoïde, pour utilisation selon la revendication 1, dans lequel est un traitement modificateur de la maladie par opposition à un traitement symptomatique.

3. Tétrahydrocannabivarinne (THCV), un phytocannabinoïde, pour utilisation selon l’une quelconque des revendications précédentes, en association avec un ou plusieurs autres médicaments antidiabétiques ou médicaments antiobésité.

4. Tétrahydrocannabivarinne (THCV), un phytocannabinoïde, pour utilisation selon la revendication 3, dans lequel le médicament antidiabétique supplémentaire est la metformine.

5. Tétrahydrocannabivarinne (THCV), un phytocannabinoïde, pour utilisation selon la revendication 3, dans lequel le médicament antidiabétique supplémentaire est de la classe des sulfonylurées des médicaments antidiabétiques.

6. Tétrahydrocannabivarinne (THCV), un phytocannabinoïde, pour utilisation selon l'une quelconque des revendications précédentes, dans lequel le THCV est un cannabinoïde de synthèse ou un phytocannabinoïde isolé.

7. Tétrahydrocannabivarinne (THCV), un phytocannabinoïde, pour utilisation selon l'une quelconque des revendications 1 à 5, dans lequel le THCV est présent sous forme d'extrait d'une plante de cannabis.

8. Tétrahydrocannabivarinne (THCV), un phytocannabinoïde, pour utilisation selon la revendication 7, dans lequel l'extrait d'une plante de cannabis est une substance phytothérapeutique.

9. Tétrahydrocannabivarinne (THCV), un phytocannabinoïde, pour utilisation selon la revendication 7 ou 8, lequel est sensiblement exempt des phytocannabinoïdes tétrahydrocannabinol (THC) et/ou canabidiol (CDB)

10. Tétrahydrocannabivarinne (THCV), un phytocannabinoïde, pour utilisation selon l'une quelconque des revendications précédentes, dans lequel la dose thérapeutiquement efficace de THCV varie entre 1 et 2000 mg.

Figure 1.

**Photograph of stained pancreatic islet cell before treatment.**

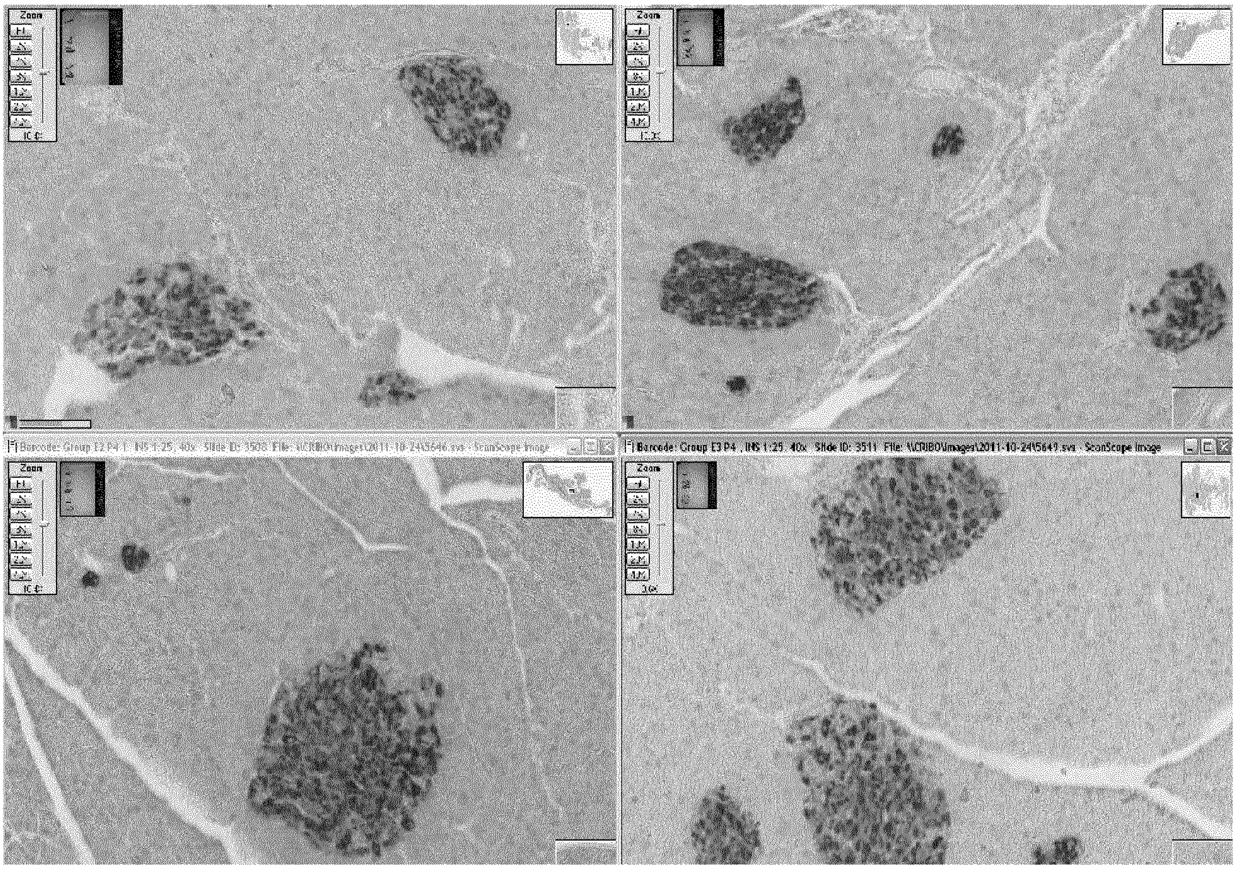

**Figure 2.**

**Photograph of stained pancreatic islet cell in vehicle treated diabetic mouse.**

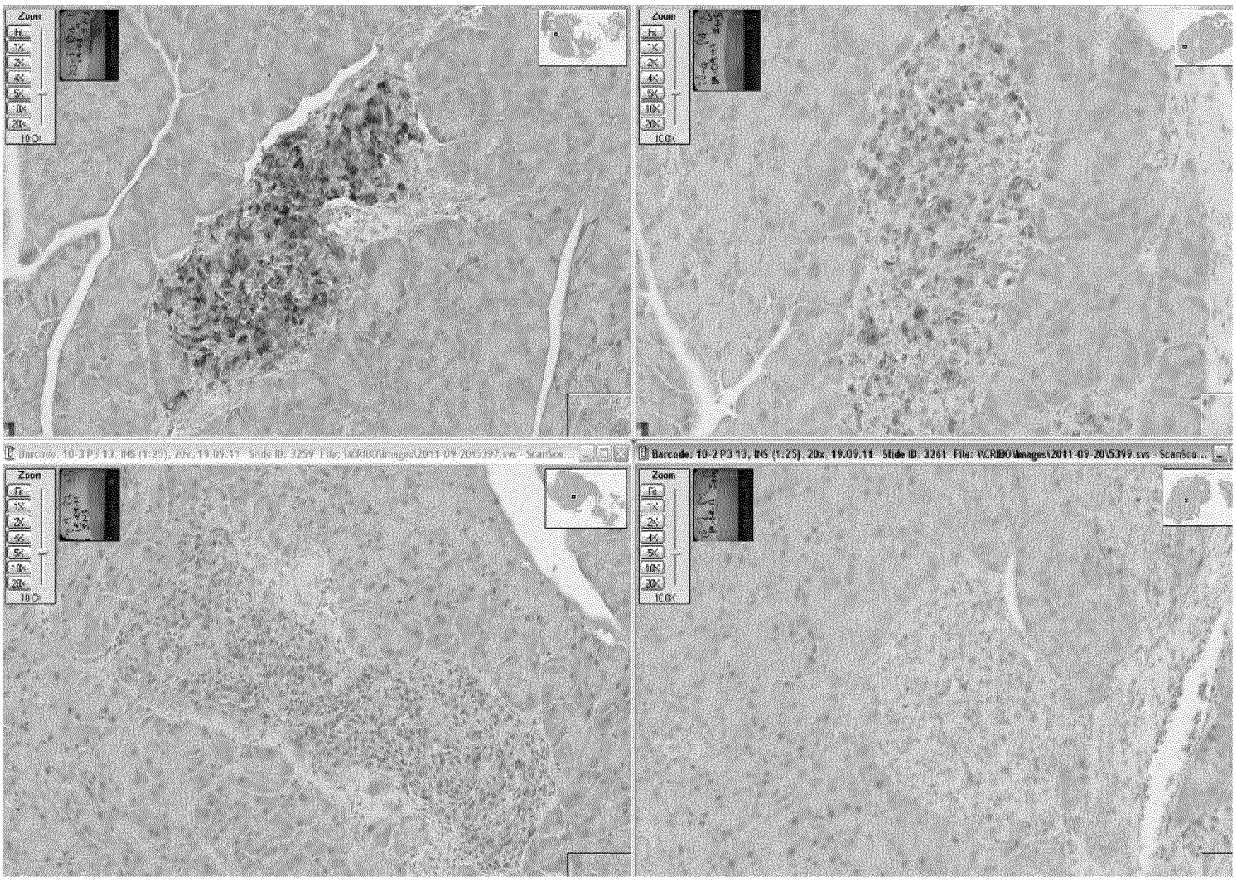

**Figure 3.**

**Photograph of stained pancreatic islet cell in AM251 treated diabetic mouse.**

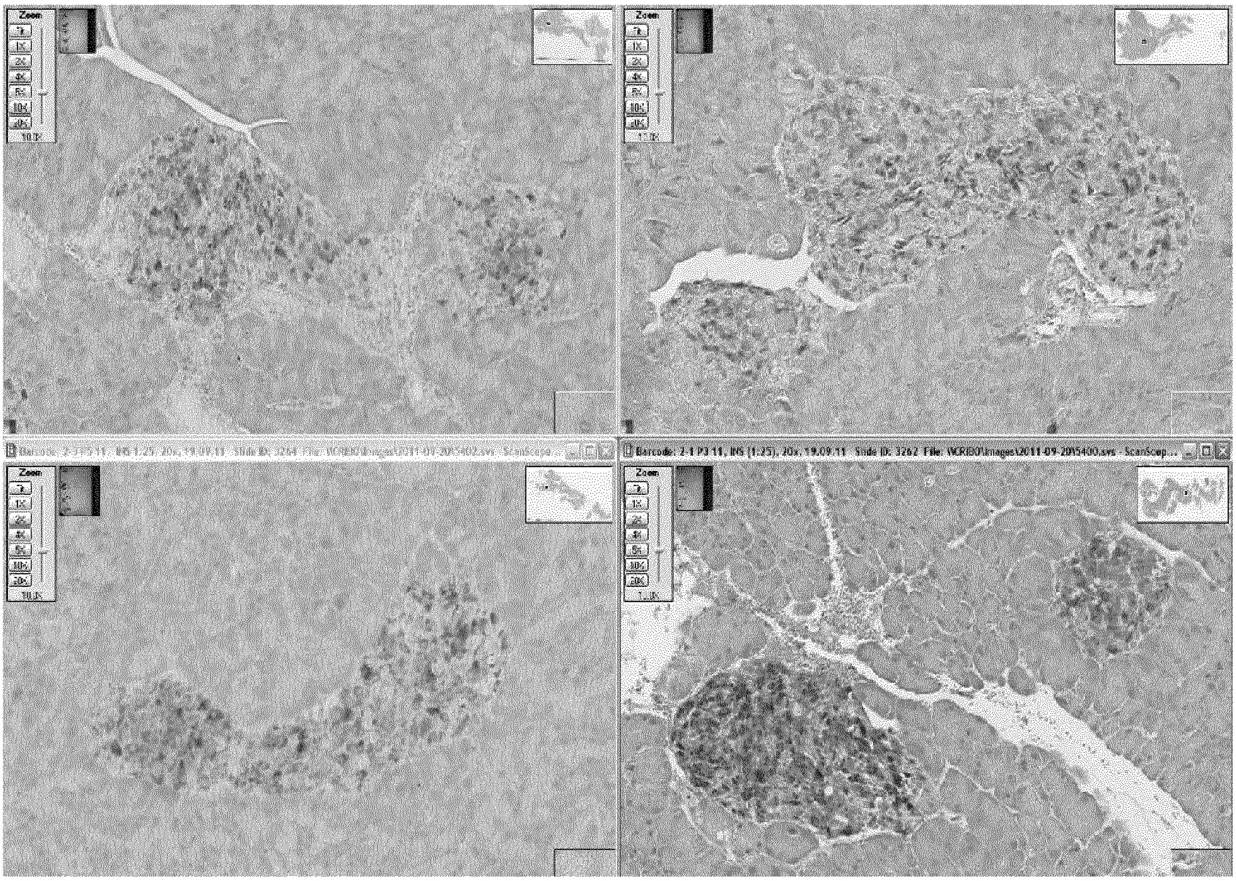

**Figure 4.**

**Photograph of stained pancreatic islet cell in CBD treated diabetic mouse.**

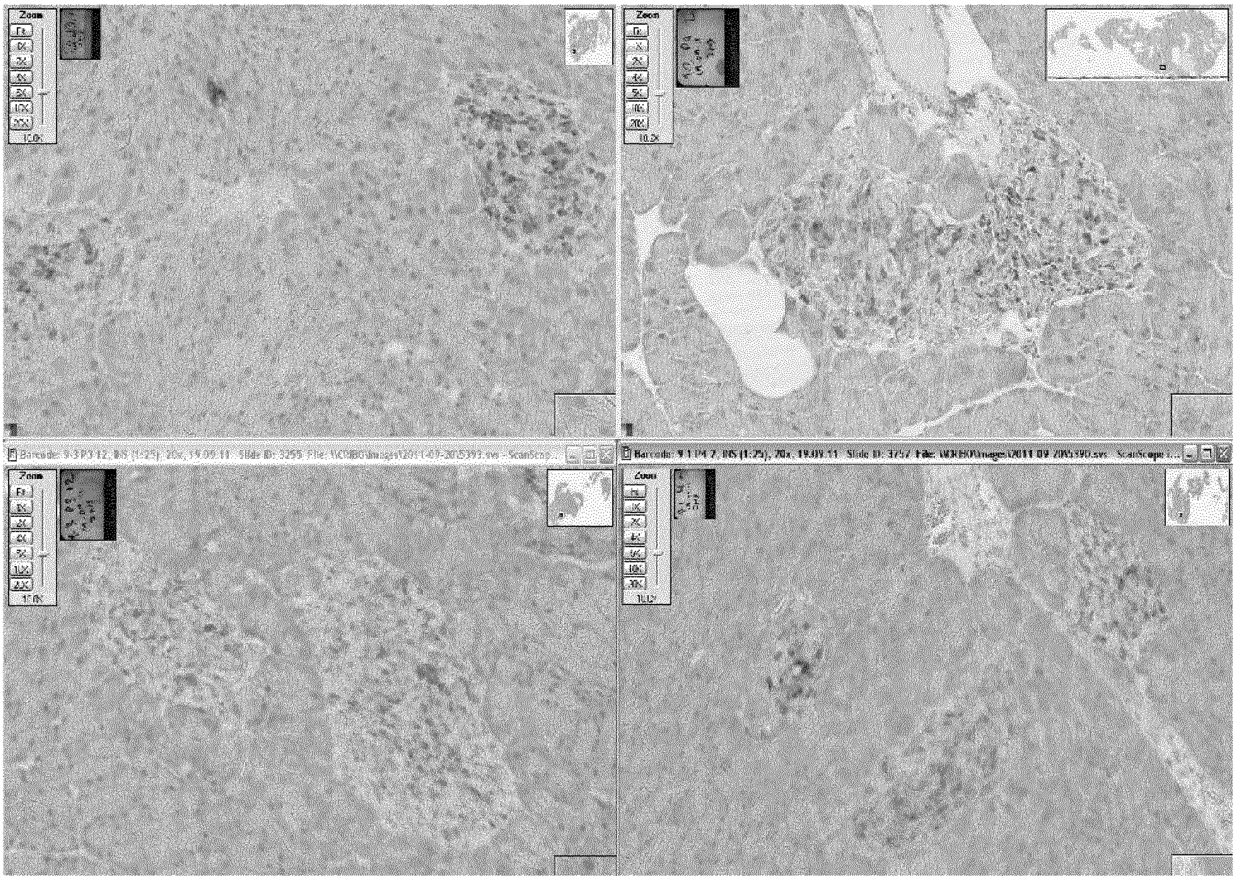

**Figure 5.**

**Photograph of stained pancreatic islet cell in THCV treated diabetic mouse.**

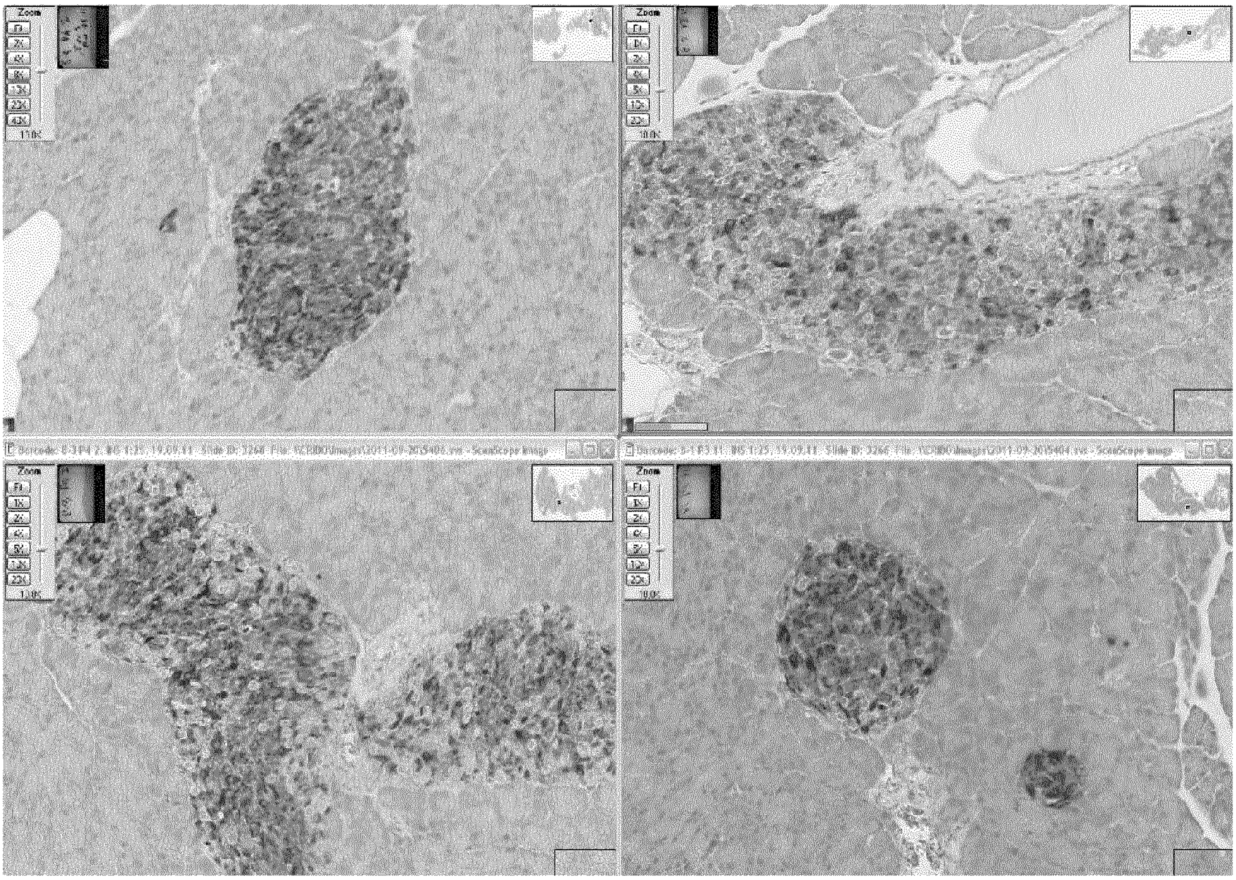

**Figure 6.**

**Bodyweight change of animals over the study period**

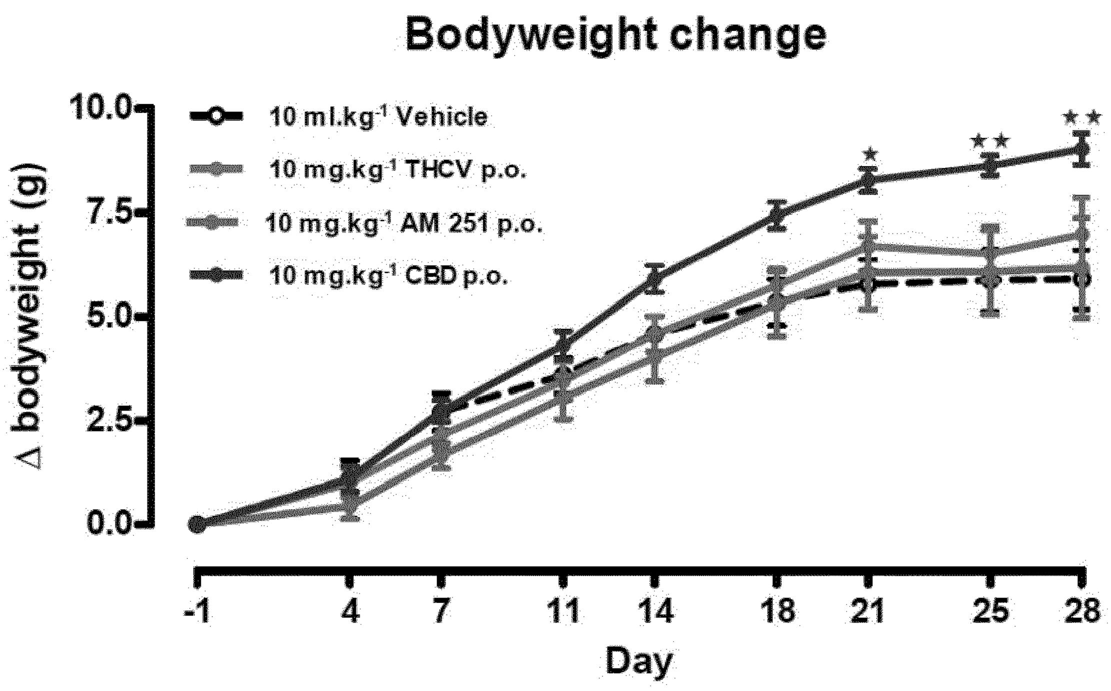

**Figure 7.**

**Blood glucose concentration over a 24 hour period**

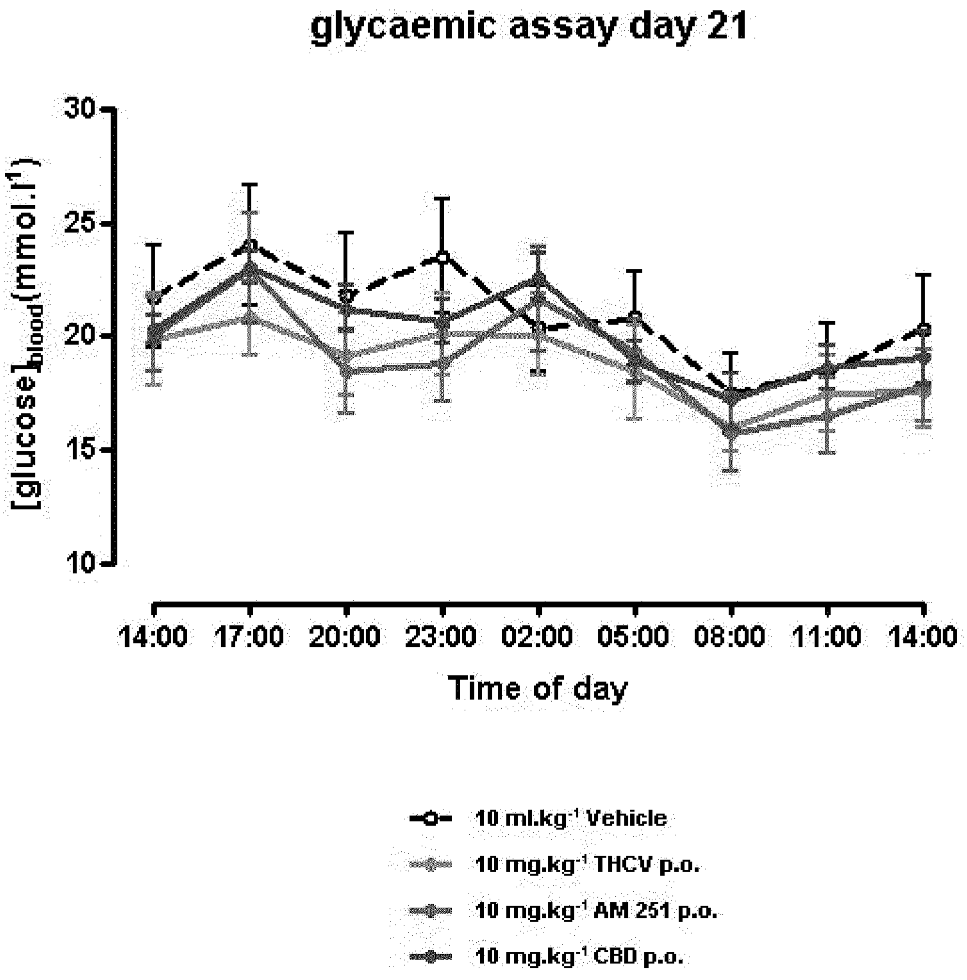

**Figure 8.**

**Pancreatic islet beta cell mass in animals at the end of the study period**

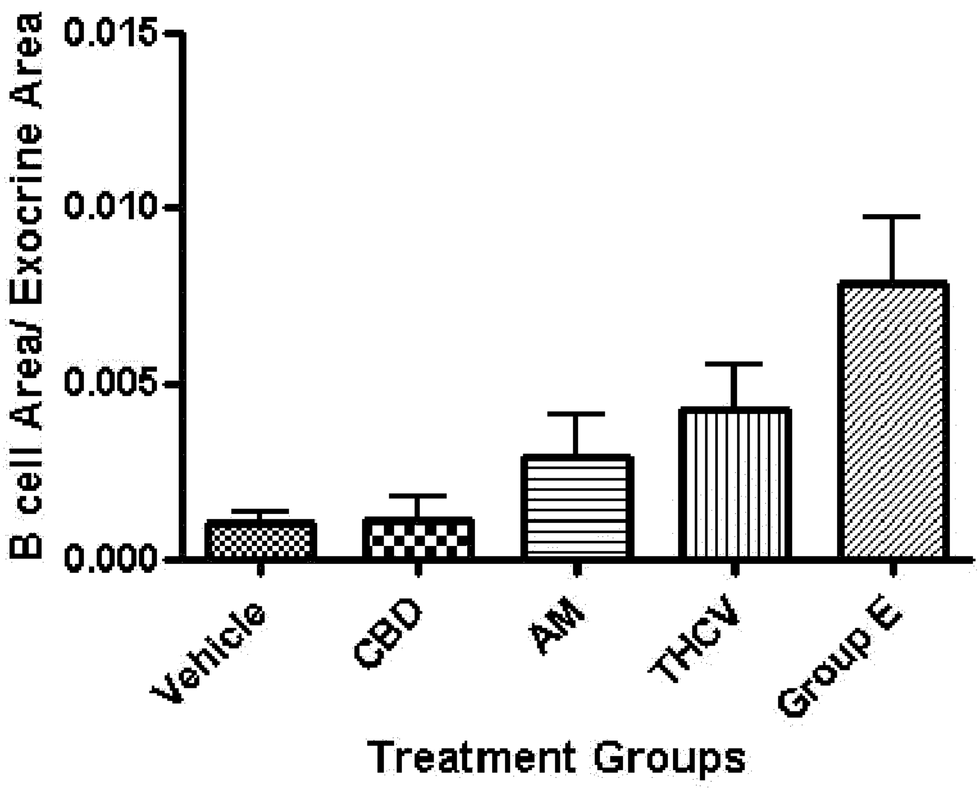

Figure 9

Blood glucose concentration at Day 0

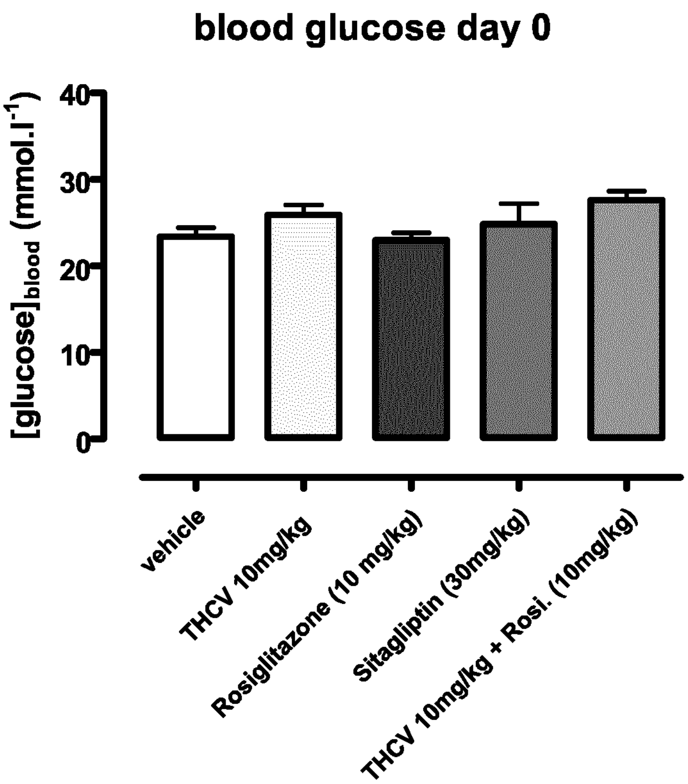

**Figure 10**

**Blood glucose concentration at Day 7**

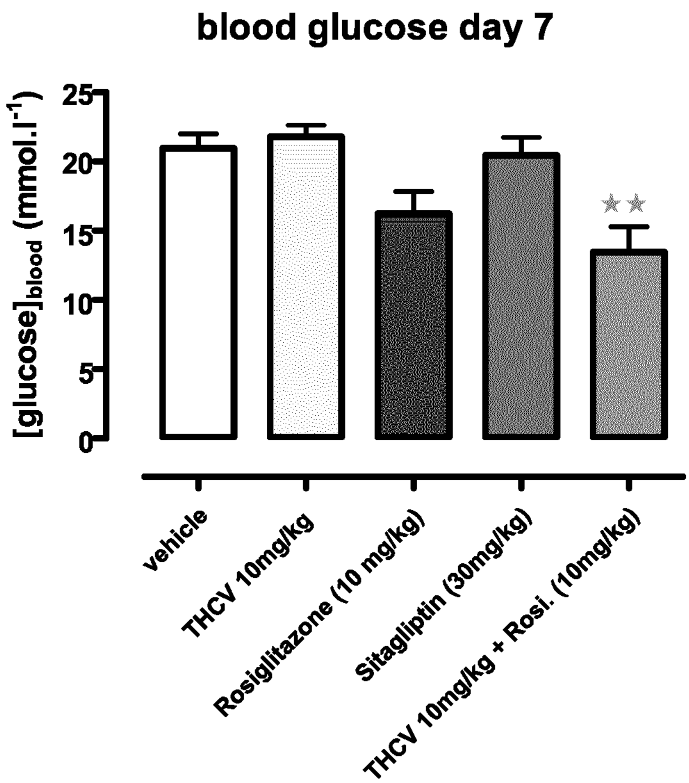

**Figure 11**

**Blood glucose concentration at Day 14**

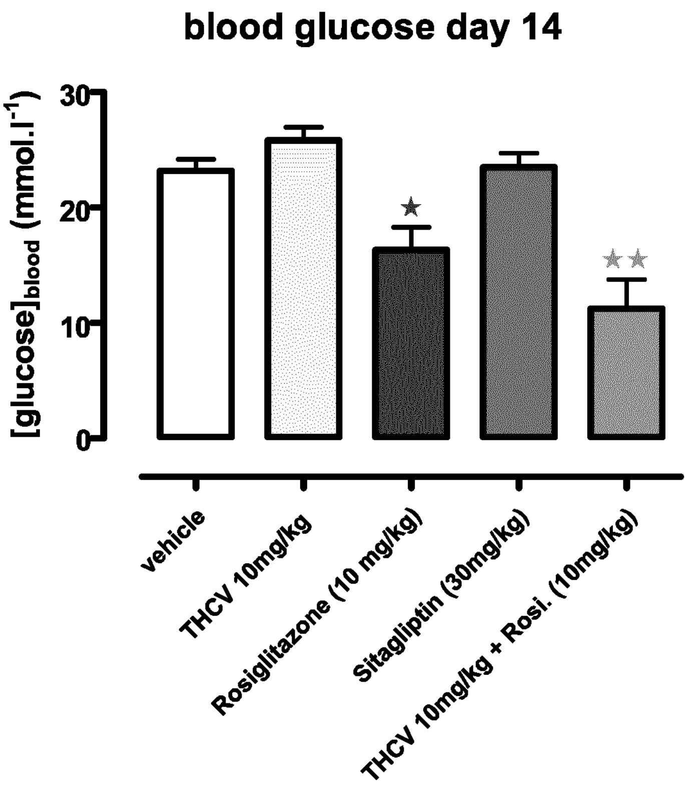

Figure 12

Blood glucose concentration at Day 23

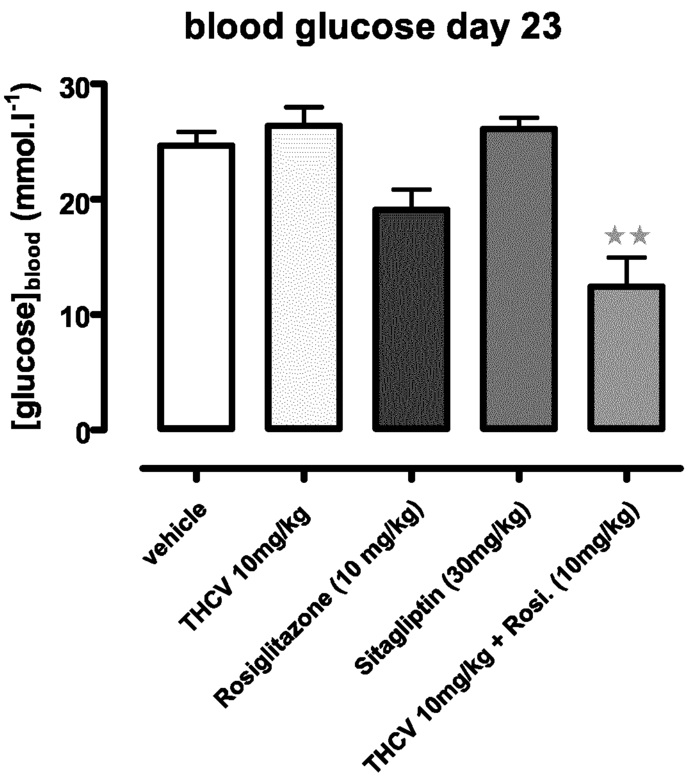

**Figure 13**

**Blood glucose concentration during an oral glucose tolerance test – area under the curve.**

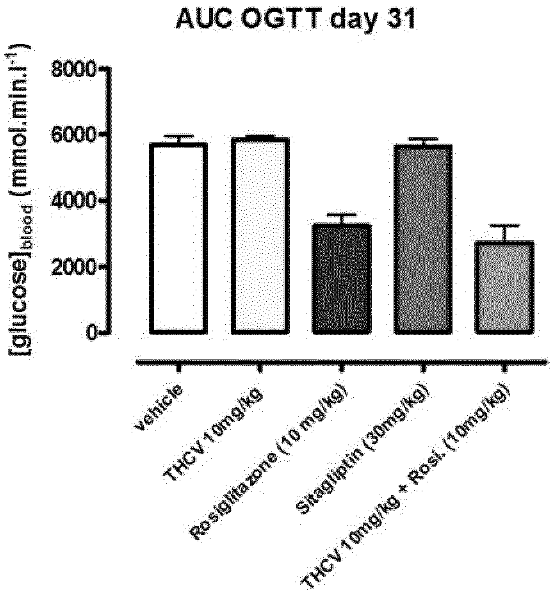


| | | | | |
|---|---|---|---|---|
| Bonferroni's Multiple Comparison Test | Mean Diff. | t | Significant? P < 0.05? | Summary |
| vehicle vs THCV 10mg/kg | -156.6 | 0.3226 | No | ns |
| vehicle vs Rosiglitazone (10 mg/kg) ) | 2434 | 5.190 | Yes | *** |
| vehicle vs Sitagliptin (30mg/kg) ) | 45.23 | 0.09317 | No | ns |
| vehicle vs THCV 10mg/kg + Rosi. (10mg/kg) ) | 2950 | 6.290 | Yes | *** |
| Rosiglitazone (10 mg/kg) vs THCV 10mg/kg + Rosi. (10mg/kg) ) | 516.0 | 1.100 | No | ns |

**Figure 14**

**Mean change from baseline of the concentration of insulin during an oral glucose tolerance test in the THCV treated group at Visit 5**

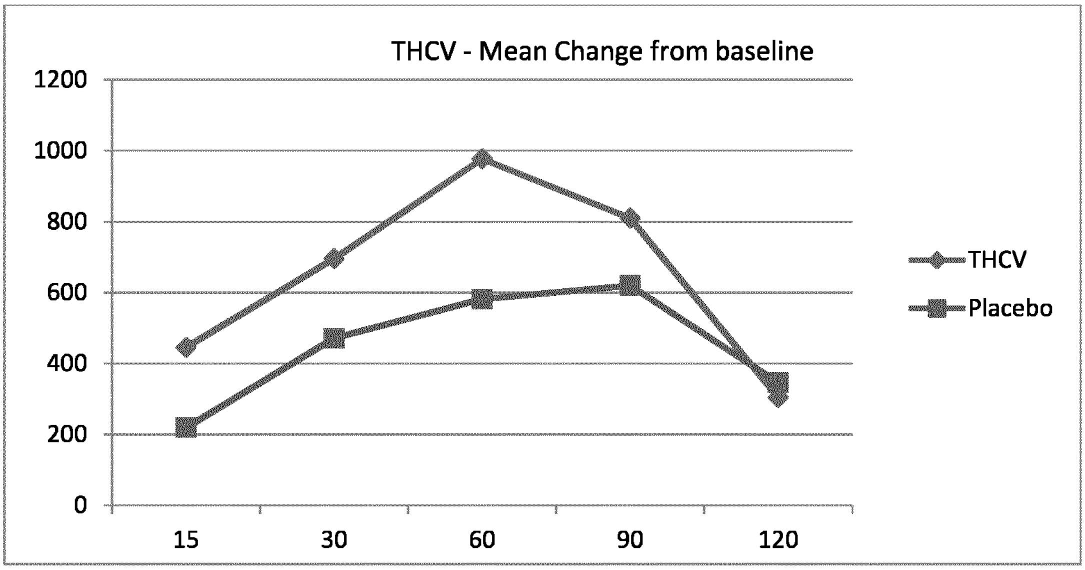

**Figure 15**

**Mean change from baseline of the concentration of blood glucose during an oral glucose tolerance test in the THCV treated group at Visit 5**

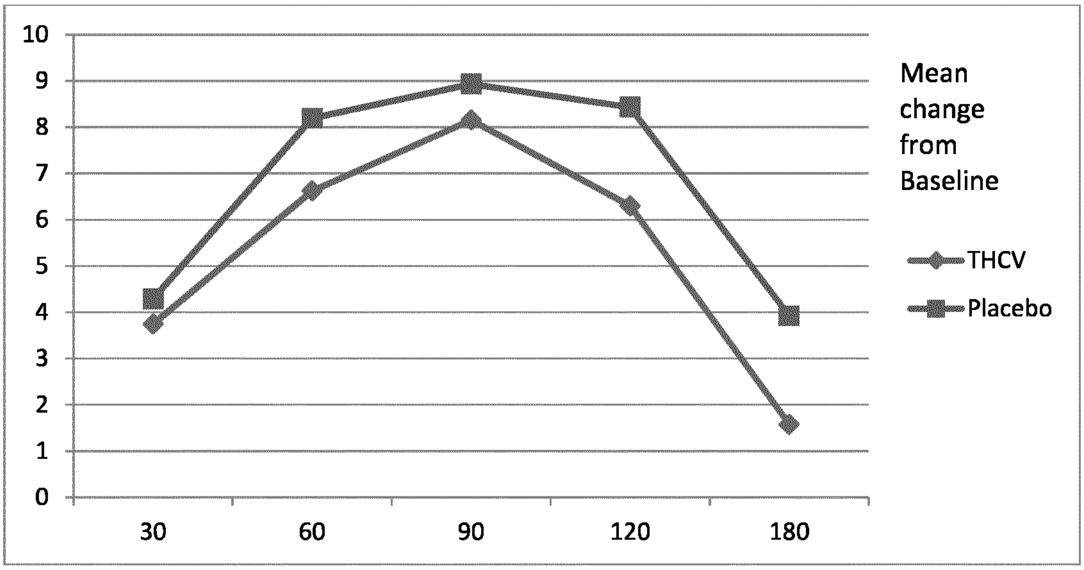

**Figure 16**

**Change from Baseline in HOMA2 Insulin Sensitivity**

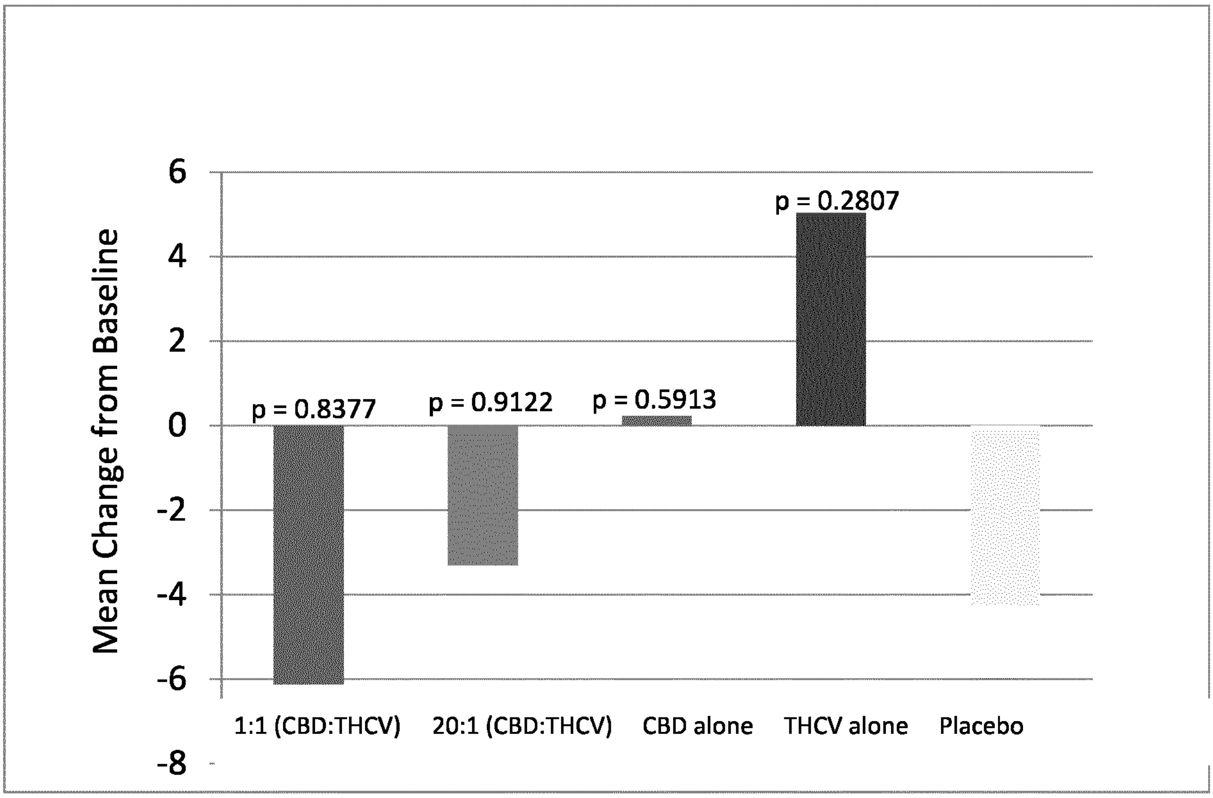

**Figure 17**

**Change from Baseline in HOMA2 Beta Cell Function**

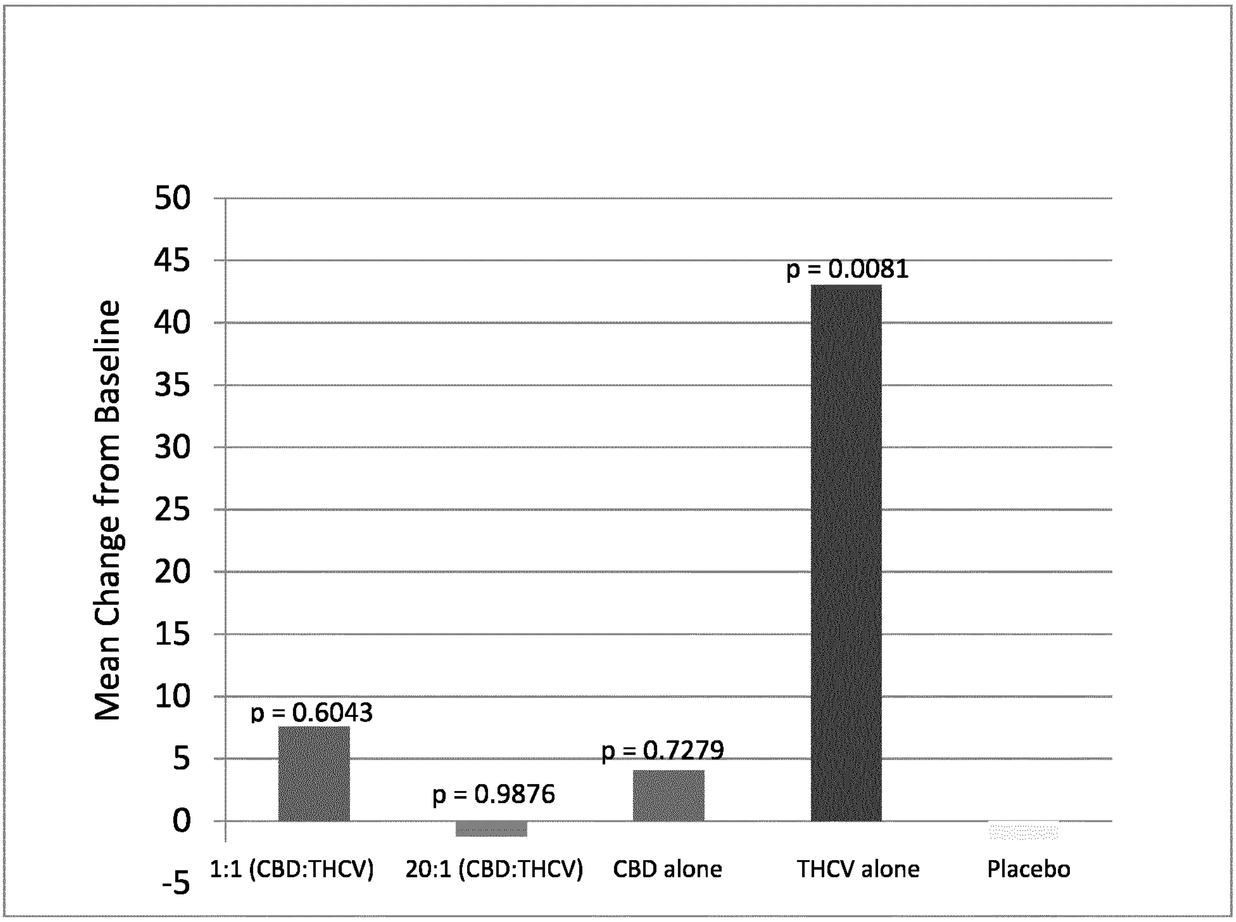

**Figure 18**

**Change from Baseline in Mean serum Glucagon-Like Peptide 1 (GLP-1) Levels (pg/mL)**

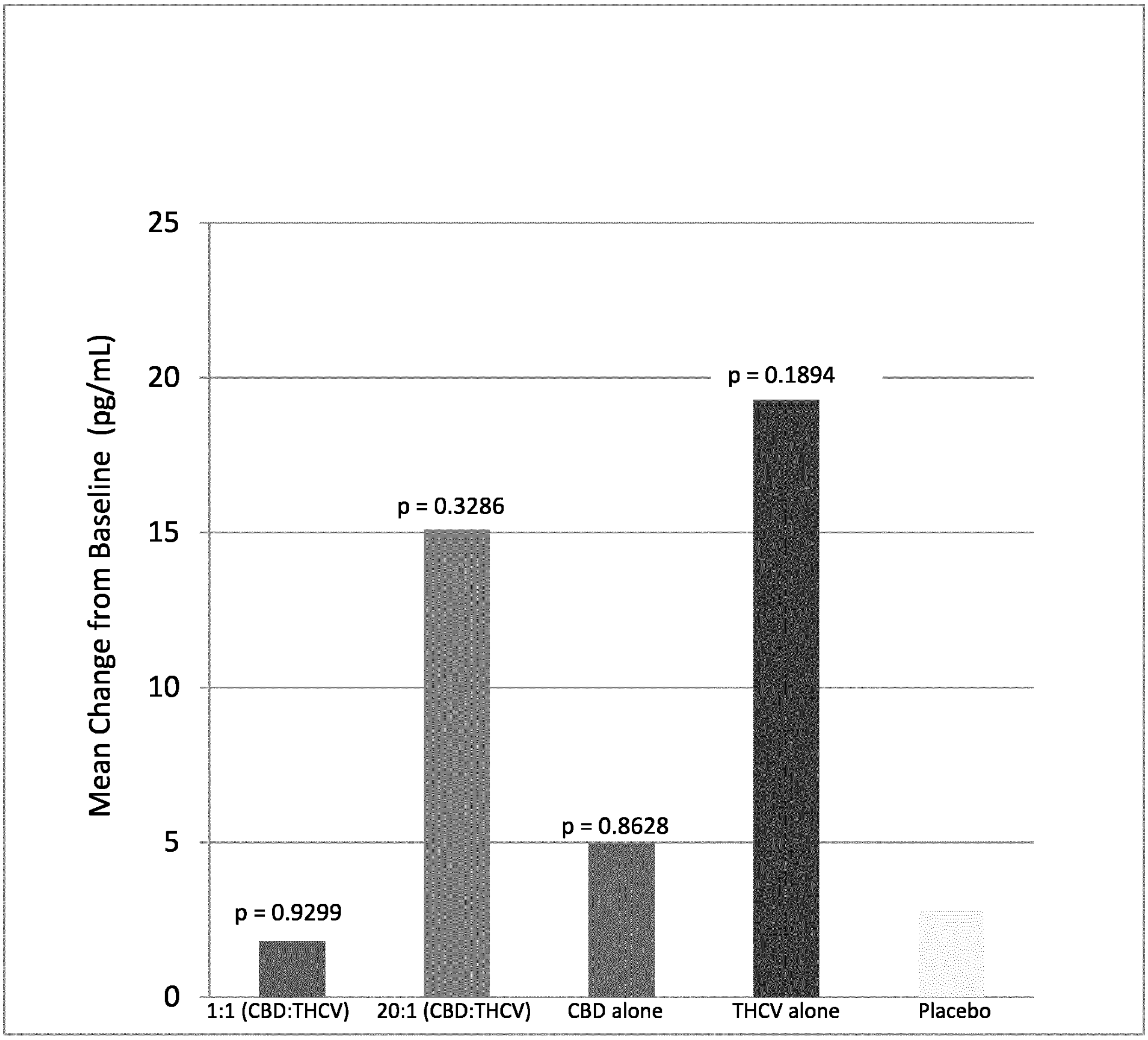

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 2434097 A
- US 20070099987 A
- WO 2009007697 A
- WO 2009093018 A
- WO 2007032962 A
- WO 2006054057 A


### Non-patent literature cited in the description


- Guidance for Industry Botanical Drug Products Draft Guidance. US Department of Health and Human Services, Food and Drug Administration Centre for Drug Evaluation and Research, August 2000